# EUROPEAN PATENT SPECIFICATION

(11) **EP 0 470 192 B1**
(45) Date of publication and mention of the grant of the patent: **08.10.1997**
(21) Application number: 90908003.8
(22) Date of filing: 16.04.1990
(51) Int. Cl.: C12N 9/96, G01N 33/535, G01N 33/543, G01N 33/546, G01N 33/553

(54) **IMPROVED IMMUNOASSAY INCLUDING LYOPHILIZED REACTANT MIXTURE**
VERBESSERTES IMMUNOTESTVERFAHREN ENTHALTEND EINE MISCHUNG VON LYOPHILISIERTEN REAGENZIEN
IMMUNO-ANALYSE AMELIOREE COMPRENANT UN MELANGE DE REACTIFS LYOPHILISE

(30) Priority: 28.04.1989 US 344575
(43) Date of publication of application: 12.02.1992
(73) Proprietor: Hygeia Sciences, Inc., Newton Massachusetts 02160-1432 (US)
(72) Inventor: COLE, Francis, X., Stow, MA 01775 (US)
(74) Representative: Bizley, Richard Edward
(86) International application number: PCT/US90/02064
(87) International publication number: WO 90/13637

(56) References cited:
- EP-A- 0 111 216
- EP-A- 0 140 489
- EP-A- 0 365 685
- WO-A-87/00196
- AU-B- 3 026 889
- DE-A- 2 712 044
- US-A- 4 853 335
- US-A- 4 859 612
- US-A- 4 931 385
- PATENT ABSTRACTS OF JAPAN vol. 9, no. 323 (p-414) (2046) 18 December1985 & JP-A-60 149 972 (YATORON K.K.) 7 August 1985

## Description

The present invention relates to immunoassays and immunoassay techniques for the detection of bindable substances, such as antibodies and antigens, and in particular to improved lyophilized reactant mixtures for use therewith. More particularly, the invention relates to improved materials and methodology for use in connection with the metal sol capture immunoassay procedures and kits disclosed in co-assigned and co-pending United States applications, Serial No 105,285, filed October 7, 1987 and Serial No 177,114, filed April 4, 1988. The disclosures of the US patent applications have been published as follows:
- 105,285:: issued as US 4859612 on 22 August 1989
- 117,114:: corresponding Australian application No 30268/89 published 5 October 1989.

The entireties of the disclosures of the '285 and '114 applications are also hereby specifically incorporated by reference. Additionally, the disclosure of the '285 application provides an excellent description of prior developments in the field of diagnostic procedures based on immunochemistry and reactions.

### Description of Prior Activities and Developments

US 4859612 describes a process for the determination and detection of an immunologically reactive analyte in an aqueous sample.

The process involves the provision of a labelled component comprising the coupling product of an immunologically reactive substance and a metal-containing particle of a size and character to facilitate the maintenance of a generally stable, monodispersed suspension of the labelled component. Also provided is a solid phase component which comprises the coupling product of an immunologically reactive substance and a solid phase particle of a size and character to facilitate the maintenance of a generally stable suspension of the solid phase component. The solid phase component and the labelled component are mixed together and brought into contact with the sample to be analyzed so as to form a single mixed aqueous suspension containing the components and the sample to be analyzed for analyte. The immunologically reactive substances coupled to the particles are capable of binding directly or indirectly as a function of the presence of analyte to thereby form a dispersed, collectable, solid phase, metal-containing composite. The procedure includes the final step of collecting the composite and determining or detecting the analyte in the sample by evaluating, through direct visual examination, the presence of metal in the collected solid phase composite.

In accordance with the invention of US 4859612 metal sol particles having a particle size in the range of from about 50 to about 1000 Angstroms may be coated with antibodies. Such metal particles, and in particular gold sol particles with antibodies coated on their surface have already been described by M. Horisberger et al. in *Experimentia* 31, pp. 1147-1149, 15 October, 1975. Such gold particles coated with antibody or antigen are intensely coloured, either orange, red or violet, depending on particle size.

It was found that in many immunoassays which normally employ enzymes as colour formers, gold labelled immunoreactants may be directly substituted for the enzyme labelled immunoreactants without significant loss of sensitivity. This was a most important finding since gold labelled antibodies are, under the conditions described below, directly visualizable with the naked eye, whereas detection of enzyme labelled antibodies requires addition of substrate for that enzyme. Metal sol assays, and in particular gold sol assays, therefore, require one less step and one less reagent. In addition, colloidal gold particles are in general, considerably more stable than most enzyme labelled antibodies.

In accordance with the procedure of US 4859612, gold particles coated with an immunoreagent are mixed with a suspension of solid particles such as latex, silanized glass, Sepharose, Reactogel or isothiocyanate activated glass, which solid particles are also coated with an immunoreagent. Such mixed suspension is contacted by a sample containing an analyte that is bindable to either or both of the immunoreagents bound to the gold sol phase and the suspended solid phase. The gold particles are then immunospecifically linked to the solid phase (sandwich mode) or anticipated linkage is prevented (competitive inhibition mode).

In exemplary embodiments, a suspended solid phase is collected by sedimentation, centrifugation, or entrapment on and within a porous capture matrix. The assay results are obtained by direct visual examination of the captured solid phase. If gold sol particles are employed, then the captured solid phase will be either intensely red, pink or essentially colourless, depending on the presence of the analyte and assuming that the solid phase is itself colourless in the absence of the gold sol particle.

The metal sol particles to be used may be prepared by methodology which is known. For instance, the preparation of gold sol particles is disclosed in an article by G. Frens, *Nature*, 241, 20-22 (1973). Additionally, the metal sol particles may be metal or metal compounds or polymer nuclei coated with metals or metal compounds, all as described in US patent 4313734. In this regard, the metal sol particles may be of platinum, gold, silver or copper or any number of metal compounds which exhibit characteristic colours.

The solid phase particles may comprise any one of a number of known, water dispersible particles, such as, for example, the polystyrene latex particles disclosed in U.S. Pat. No. 3,088,875. Such solid phase materials simply consist of suspensions of small, water-insoluble articles to which proteins, such as the immunologically reactive substances of the present invention, are able to bind. Suitable solid phase particles are also disclosed, for example, in U.S. Pat. Nos. 4,184,849; 4,486,530; and 4,636,479.

The solid phase particles useful in connection with the invention may comprise, for example, particles of latex or of other support materials such as silica, agarose, glass, polyacrylamides, polymethyl methacrylates, carboxylate modified latex and Sepharose. Preferably the particles will vary in size from about 0.2 microns to about 10 microns. In particular, useful commercially available materials include 0.99 micron carboxylate modified latex (Polysciences), cyanogen bromide activated Sepharose beads (Sigma), fused silica particles (Ciba Corning, lot #6), isothiocyanate glass (Sigma), Reactogel 25DF (Pierce) and Polybead - carboxylatemonodisperse microspheres (Polysciences). Such particles may be coated with a layer of immunologically reactive substances coupled thereto in a manner known per se in the art to present the solid phase component.

Either monoclonal or polyclonal antibodies may be employed, in accordance with the teaching of US 4859612, for detecting or determining specific antigens. Monoclonal antibodies which are useful may be prepared in accordance with the discovery of Milstein and Kohler. Monoclonal antibodies which are particularly desirable in accordance with the present invention may be prepared using the polyethylene glycol (PEG) promoted hybridization method of Gefter, Margulies and Scharff reported in *Somatic Cell Genetics,* Vol. 3, No. 2, 1977, pp. 231-236. Suitable antibodies useful in detecting human choriogonadotropin (hCG) and human leutenizing hormone (hLH) may be prepared using a slightly modified Gefter et al. procedure involving immunisation of strain A mice (Jackson Laboratories, Bar Harbor, Maine) with either hLH or hCG utilising any one of a variety of known immunisation schedules, antigen doses and modes of antigen presentation. Such procedures induce the production of antigen reactive serum antibodies capable of successfully producing hybridoma cell lines. In particular, hybridomas were prepared by fusing Sp6 mice lymphocytes from immune mice with SP2/0-Agl4 cells, see M. J. Shulman and G. Kohler, *Nature,* Vol. 274, pp 917-9 (1978), via the polyethylene glycol catalysed cell fusion procedure of Gefter et al. Hybridoma tissue culture supernatants were initially assayed by means of a solid phase antigen binding immunoassay in accordance with the method of Klinman et al. (Klinman, N. R., Pickard, A. R., Sigal, N. H., Gearhart, P. J., Metcalf, E. S. and Pierce, S. K., *Ann. Immunol.* (*Inst Pasteur*), Vol 127 C, pp 489-502 (1976)).

After assaying and screening using conventional procedures, hybridoma cell lines for producing antibodies having appropriate binding efficiencies and specificities were identified. The antibodies produced using such hybridoma cell lines are identified, for convenience, using the terminology 2B2 for a hCG specific antibody, LH26 for a hLH specific antibody and HCG/KLH/2G9 for an antibody which reacts specifically with both hLH and hCG. The 2B2 antibody binds selectively to one discrete binding site on the hCG antigen molecule while the HCG/KLH/2G9 antibody binds selectively to a separate, discrete binding site on the hCG molecule. Likewise, the LH26 and HCG/KLH/2G9 antibodies bind selectively to separate, respective, discrete binding sites on the hLH molecule.

US 4859612 states that the optimal conditions for coating proteins onto gold sol particles differ from protein to protein and from batch to batch of the gold sol particles. Such conditions must in general be determined empirically. To determine the optimum conditions, proteins to be absorbed to the gold sol particles are first exhaustively dialysed at room temperature against 2 millimolar (mM) borate-10 mM azide solution having a pH of 8.0. Prior to coating, the dialysed preparation is filtered through a 0.20 micrometer (um) Millex GV filter (Milipore, Bedford, Massachusetts). The protein concentrations are determined spectrophotometrically using an extinction coefficient of 1.4. To determine the quantity of protein needed for optimum coating of gold sol particles, pH and protein concentration variable isotherms are constructed. The method of Goodman, Hodges, Trejdosiewicz and Livingston, *Journal of Microscopy,* Vol. 123, pt. 2, August, 1981, pp. 201-213, is used with some modifications, as indicated hereinafter. The effect of pH on the absorption of protein on gold particles is examined in a series of eleven different buffers having pHs ranging from 2.3 to 11.0. The buffers used, in order of increasing pH, are tris, sodium phosphate, sodium carbonate, citric acid, boric acid, barbital, piperazine, morpholine, lactic acid, sodium salicylate and phthalic acid. For spectrophotometric analysis, 270 microliters (ul) of gold sol is added to 30 ul aliquots of 70 mM buffer containing 10 mM concentration of azide. These solutions are vortexed and allowed to remain at room temperature for 15 minutes. 60 ul of a 50 microgram (ug) per ml protein solution is then added while vortexing. After standing for one hour at ambient temperature, 60 ul of a 10% sodium chloride solution is added. The absorbance is measured at the maximum absorbance (525 nanometers(nm)) for the unflocculated sol. The reaction is also scored visually since the sol is originally orange/red and the colour changes to blue/grey upon flocculation. Exploiting this property of colloidal gold, the optimum buffer is selected in accordance with the Goodman et al. procedure. A protein concentration isotherm may be determined in a similar manner using concentrations ranging from 1 mg per ml to 3.9 mg per ml. In this way, an optimum buffer and adsorptive protein concentration may be selected for each combination of protein material and gold sol batch. The effects of over adsorption of protein may also be explored using preparations of probes at protein concentrations above and below the optimal coating levels.

In an exemplary procedure for making antibody-coated latex particles, 1.0 ml of 0.99 u carboxylate modified latex (Poly-sciences) and 20 ml of a 0.15 M NaCl solution are placed in a centrifuge tube. 0.75 ml of a water solution of 1-ethyl-3-(3-dimethylaminopropyl) carbodiimide hydrochloride at a concentration of 100 mg/ml is added to the centrifuge tube and the admixture is stirred for 20 minutes. Thereafter, 11 ml of a 0.15 M NaCl solution is added and this mixture is sedimented at 38,000 G for 10 minutes. The supernatant is discarded and the pelleted latex particles are resuspended in 20 ml of the 0.15 M NaCI solution. The suspension is re-centrifuged as above and the pellet finally resuspended in 4 ml of the 0.15 M NaCI solution. 2 ml of this latex suspension, 1.5 ml of the 0.15 M NaCl solution and 1.5 ml of a solution containing hCG specific antibody (designated above by the designation 2B2) at a concentration of 1.678 mg/ml, are mixed in a 15 ml plastic test tube. The tube is capped and the mixture is incubated with end over end stirring on a Labquake rotator for 16 hours at 22°C. Thereafter, 1.0 ml of a I M lysine solution having a pH of 7.5 is added and the mixture is stirred for an additional 30 minutes. The antibody coated latex is then washed four times with 30 ml of 0.15 M NaCl solution with repeated pelleting as set forth above. The final pellet is resuspended in 2 ml of 0.15 M NaCl solution containing 1 mg/ml of BSA.

A porous matrix capture format may be used to collect the solid phase component. In the porous matrix capture format, the dispersed, collectible, solid phase, metal containing composite, irrespective of the solid phase component it contains, is captured on the surface of and in the interstices and pores of the porous matrix. To facilitate the matrix capture process in the laboratory, a self contained flow device has been constructed which serves to hold the capture matrix in tight contact with an absorbent so that the flow of liquid through the capture matrix is spontaneous and does not require a vacuum or external pressure source. The absorbent medium which was found to be most useful in the laboratory was a Transorb (American Filtrona) unit consisting of a cellulosic plug. It was found that when a separator, was placed between the capture matrix and the absorbent, complete separation between the absorbed fluid phase and the captured and collected solid phase on the porous matrix is ensured.

US5849612 describes that a number of separator layers had been evaluated and that it had been found that the same may be composed of a glass fibre layer (Whatman), blotter paper (Gillman), or a porous plastic layer (Porex or Pellon) with essentially the same results. Likewise, a considerable number of capture matrices had been evaluated and found to be suitable. Glass fibre filters (Whatman GF/A), regenerated cellulosic membranes (Schleicher and Schuell) and microporous membranes (Millipore MF series membranes HAWP, SSWP, SMWP and SCWP with pore sizes of 0.45, 3, 5 and 8 microns respectively) had all been successfully utilised for capturing and collecting solid phase, metal-containing composites, in accordance with the present invention.

AU-B-30268/89 is applicable to assay procedures IV (f) and (g) of US 4859612. These procedures are as follows:

IV(f): (f) A porous matrix capture assay for hCG (human choriogonadotropin) ia conducted as follows. In this procedure 2 assay tubes are prepared, each containing a mixture of 50 ul of hCG specific antibody coated latex particles, 300 ul of antibody coated gold sol particles and 300 ul of PBS-BSA buffer. One of the tubes also contains 100 ul of a standard solution containing 50 mIU of hCG per milliliter, while the other also contains 100 ul of a standard solution containing no hCG. The resultant admixtures, each containing a dispersed solid phase component comprising the antibody coated latex particles and a labelled component comprising the antibody coated gold probe particles, have orange/red colorations. The mixtures in the tubes are incubated at room temperature for 10 minutes during which time the colour in the tube does not change. The samples, are poured onto a glass fibre filter (Whatman GF/A) matrix mounted in a self contained flow device as described below. A distinctively pink coloured visually apparent spot appears on the matrix at the point contacted by the solution containing the hCG. No such spot appears where the matrix is contacted by the solution containing no hCG. The spotted matrix is shaded from light for four days and the pink spot retains its original intensity. Similar spots have been retained in notebooks for 6 to 12 months and are still highly recognisable.

(g) Another porous matrix capture assay for hCG is conducted using a procedure identical to that of Example IV(f), except that a different gold probe is used. In this case, the solutions initially have a dark purplish coloration and the coloured spot which develops at the point where the solution containing the hCG contacts the capture matrix has a pink-purplish colour. Otherwise the rcsults are the same as obtained in Example IV(f).

According to AU-B-30268/89, in the past, false positive readings had sometimes been the result when procedures such as those disclosed in the '612 US patent were used to test for hCG or hLH. The exact causation for such false readings is not completely understood; however, it is believed that the false positive readings are the result of several separate phenomena. Firstly, human urine contains certain sedimentation which may tend to clog the pores of the collection filter and prevent, or at least inhibit flow of the aqueous reaction phase through the filter. Thus, gold particles carrying antibodies which have not reacted to form an immunocomposite may be prevented from flowing through the filter and as a result will be non-specifically present on the filter even after washing to exhibit the pinkish to purplish coloration that is the inherent characteristic of the gold sol particle. Secondly, collection filter membranes of the type utilised in connection with the present invention sometimes have a tendency to non-specifically bind unreacted antibody so as to retain unreacted gold sol particles on the collection membrane. Thirdly, certain substances in urine may act to increase the tendency of the collection filter element to non-specifically bind unreacted immunoreactive substances. Additionally, the pinkish to purplish environment that surrounds the test materials and reaction phase may provide an optical impression that persists to cause an uncoloured collection filter to appear slightly pink to an untrained eye, even after washing, and thus the test may provide a false appearance of being slightly positive or at least an appearance that is confusing.

The Australian patent provides an improvement of the prior art process which comprises treating the urine sample to be analyzed for analyte to remove undesirable contaminants therefrom without affecting the analyte content thereof; coating the filter element with a blocking agent effective to reduce non-specific binding of unreacted immunochemically reactive substances; including a sufficient amount of a colour forming material in the aqueous suspension to provide the suspension and initially the collected composite with a coloration that is capable of masking the colour of the coloured particles; and thereafter removing the colour of the colour forming material from the collected composite. Through the use of the improved process thus described, the flow of aqueous phase media and unreacted components through the filter element and direct visual evaluation of the results of the assay are each facilitated, whereby false positive readings of colour on the filter element are minimised.

In accordance with the more specific aspects of the invention, the coloured particle may comprise a gold sol particle, and the colour forming material may provide the suspension with a green or blue coloration. In accordance with another specific aspect of the invention, the urine sample may be treated to remove undesirable contaminants by contacting the sample with a material having hydrophilic characteristics dispersed on the surface of a porous carrier member. In another more specific aspect of the invention, the blocking agent may comprise polyvinyl pyrrolidone.

The material having hydrophilic characteristics may be polyethylene glycol p-isooctylphenylether.

In accordance, therefore, with certain specific procedures disclosed in US 4859612 and in AU-B-30268/89, antibody-gold sol particle conjugates and antibody-solid phase particle conjugates are dispersed together in an aqueous system containing human urine. In accordance with certain specific procedures antibody-enzyme conjugates are dispersed in an aqueous system containing human urine. When such materials are to be utilised for testing at a place or time that is remote from the place or time where the reactants are prepared, suitable methodology must be employed to ensure the preservation of the reactants and the physical retention of the characteristics thereof which permit the same to be readily dispersed in an aqueous system, particularly one which is made up predominately of human urine.

Lyophilization, or freeze drying as the procedure may be more commonly known, may be used to preserve the ingredients and components for use in procedures such as those which are utilised in the conduct of immunoassays. It is important, however, that the lyophilization procedure results in a homogenous distribution of the components of a multi-component mixture and that the lyophilized mixture remains homogenous and in a readily utilizable form during the storage period. In particular it is important that the lyophilized, solid state materials are readily dispersible upon contact with the test materials to immediately produce a solution and/or dispersion wherein the various components and ingredients are essentially either in solution or in a monodispersed condition.

It is sometimes advantageous to conduct an immunoassay procedure in the presence of binding enhancing agents to enhance formation of an immune complex and/or surfactants (or detergents) which operate to suppress non-specific interactions. Generally speaking, the binding enhancing agents and the surfactants comprise organic materials that are liquid at room temperature, and such materials should, for optimum benefits, be dispersed generally homogeneously throughout the reaction system.

As indicated above, lyophilized materials are often utilized in connection with immunoassay materials to preserve and protect the components, particularly during storage for long periods of time. And it is desirable that a single lyophilized mixture of materials be provided and which is readily dispersible in aqueous solution to supply all of the ingredients which desirably are present in the reaction system during the immunoassay period. However, it has been determined that liquid organic materials which enhance the performance of assays by performing as binding enhancing agents or as surfactants which suppress non-specific interactions are not readily incorporatable into lyophilized mixtures of materials because of their tendency to agglomerate at room temperature and disrupt the homogeneity of the lyophilized mixture. Such normally liquid organic components tend to agglomerate at room temperature to form liquid globules that not only interfere with the shelf life of the lyophilized mixture but prevent the redistribution of the lyophilized ingredients into an aqueous system when the time for conducting the immunoassay procedure has come. Accordingly, in the past it has been difficult to provide kits which include a single lyophilized mixture of materials which, upon reconstitution in an aqueous medium, supplies all of the materials which are desirably present in the reaction milieu.

### SUMMARY OF THE INVENTION

The present invention addresses the problem of providing lyophilized mixtures of materials which may be readily reconstituted in an aqueous media to present a reaction system containing all of the ingredients necessary for the conduct of an immunoassay procedure. In accordance with the present invention, the lyophilized mixture includes a component which essentially retards or inhibits the tendency of liquid organic components such as binding enhancing agents and non-specific interaction suppressing detergents to agglomerate and thus disturb the homogeneity of the lyophilized mixture. Thus, the invention provides a lyophilized mixture for use in an immunoassay procedure. The mixture comprises at least one dispersible immunoreactive component which is distributed homogeneously throughout the lyophilized mixture. The mixture further includes at least one normally liquid organic component which is also distributed homogeneously throughout the mixture, such organic component having a property which enhances the performance of the immunoassay by its presence. Additionally, the lyophilized mixture of the present invention includes a sugar comprising dextrin or trehalose, and such sugar is present in the mixture in sufficient quantity to prevent agglomeration of the organic component to thus maintain the homogeneity of the mixture to thereby facilitate storage and shelf life of the mixture and the eventual dispersion of the mixture in an aqueous medium for the conduct of an immunoassay procedure.

In accordance with the invention, the immunoreactive component may comprise an antibody conjugate such as an antibody-gold sol particle conjugate, an antibody-solid carrier particle conjugate or an antibody-enzyme conjugate.

In one form of the invention, the organic component may comprise a binding enhancing agent such as a non-ionic, water soluble polymer. Suitable materials for use as a binding enhancing agent include polyethylene glycol, polyvinyl alcohol, polyvinyl pyrrolidone and dextran, and polyethylene glycol is a particularly preferred binding enhancing agent.

In another aspect of the invention, the organic component may comprise a water soluble non-ionic surface active agent. A number of such agents are known to those skilled in the art. In accordance with the invention, a particularly preferred material comprises a polyethylene glycol p-isooctylphenyl ether compound.

Often the lyophilized mixture may include two normally liquid organic components, one of which comprises a binding enhancing agent and the other of which comprises a surfactant. And in the preferred form of the invention wherein two organic components are involved, the binding enhancing agent may comprise a polyethylene glycol and the surfactant may comprise a polyethylene glycol p-isooctylphenyl ether compound.

### DETAILED DESCRIPTION OF THE PREFERRED EMBODIMENTS

As set forth above, the present invention provides a lyophilized mixture which is readily reconstitutable in aqueous solution to supply the ingredients necessary for conducting an immunoassay procedure. The immunoassays which are particularly facilitated through the use of the present invention are those which result in the formation of a sandwich form immunocomposite. Thus, the present invention has great utility in connection with the sol capture procedures described in US 4 859 612 and AU-B-30268/89. The sol capture assays involve a first antibody coated onto a solid carrier particle and a second antibody coated onto a metal sol label particle. Such antibody-particle conjugates are then incubated with a test solution containing an analyte (usually human urine) to allow immunoreaction of the components and the resultant production of a collectible immunocomposite containing the metal sol label particles. In such assays, the labelled immunocomposite is collected and inspected visually and the color thereof resulting from the incorporation therein of the metal sol label particles is detectable with the naked eye.

One of the important aspects of the gold sol methodology is that all of the reactions necessary to form the detectable immunocomposite may proceed simultaneously in a single reaction vessel. To prepare the components, a first antibody is absorbed onto a solid carrier particle and a second antibody is absorbed onto a metal sol label particle. The test solution suspected of containing a bindable analyte is incubated with the antibody conjugates simultaneously and the immunocomposite formed if the test is positive is collected. The collected materials are visually inspected for the presence of metal sol coloration in the collected solid phase. In the conduct of such an immunoassay, it is preferable to provide an admixture of the antibody conjugates and to then admix the test solution with the mixture of conjugates. The resultant admixture may then be incubated for a prescribed period under ambient room temperature conditions between about 15°C to less than about 37°C. The mixture may then be poured onto a porous membrane filter where the immunocomposite may be collected for inspection while liquid materials and unbound labelled antibody conjugates simply flow through the membrane and are subsequently discarded. Typically the analyte is an antigen. Both the first and second antibodies may be polyclonal antibodies; however, preferably at least one of the first and second bodies is a monoclonal antibody. And in the most preferred case, the antibodies are two different monoclonal antibodies specific for respective different epitopes on the antigen molecule. In a particularly preferred form of the invention, a gold sol particle is utilized to provide a visual indication of the presence of bound components on the collection membrane.

Pursuant to the invention, the present invention facilitates the provision of kits and components that are particularly suitable for home and clinical diagnostic applications and which are implementable by unskilled users without specialized equipment. In particular the invention provides immunologic reagents which may be stored for prolonged periods without losing their reactivity, immunologic binding specificity or avidity even though the same may have been exposed to hot, humid environmental conditions during transit, or prior to use in the assay. Moreover, the reagents remain in a condition which facilitates the immediate reconstitution thereof in urine or other aqueous test samples to present complete reaction systems. Thus, the invention provides a lyophilized product mixture which may contain an antibody-metal sol particle conjugate, one of the active immunologic reagents utilized in the sol capture assay. The lyophilized product may also contain an antibody-solid phase carrier particle conjugate. In addition to the two antibody-particle conjugates, the lyophilized product mixture may preferably contain additives which are desirably present in the reaction system.

To prepare the lyophilized mixture, the desired ingredients are first all blended in an aqueous lyophilization phase. The lyophilization phase containing the conjugates and additives is subjected to freeze drying to form a powered, lyophilized (freeze dried) product. The effect of the lyophilized product mixture of the invention containing the antibody-particle conjugates is multiple. The additives to be included in admixture with the antibody-particle conjugates in the lyophilized product may be chosen for their ability to preserve the reactivity, binding specificity and avidity of the antibodies when applied to the assay even if the lyophilized product containing the antibody conjugates should be subjected to hot environmental conditions between 80°F to 120°F (27 to 49°C). In practical terms this means that antibody conjugates can be included in diagnostic kits without concern that the reactivity or immunologic binding specificity of the antibodies will be lost if the kit components are exposed to hot, humid environmental conditions. Additionally, the user may feel free to store the lyophilized product containing the conjugate at ambient conditions without the need for refrigeration and with no need for concern if environmental conditions become hot or humid.

Importantly, the lyophilized mixture has the ability to retain its homogeneity even though it contains one or more organic components which are normally liquids at room temperature and which therefore might ordinarily agglomerate subsequent to lyophilization when the lyophilized mixture is warmed to room temperature to thereby disrupt the homogeneity of the freeze dried mixture.

Lyophilized mixtures of materials facilitate the reduction of the total number of components needed for application of the procedure to home diagnostic use. Thus, a preferred diagnostic kit which incorporates the ingredients useful in connection with the sol capture procedure need include only a vial of lyophilized product containing antibody-particle conjugates and other reaction ingredients; a measuring dispenser such as an eye dropper; and an immunocomposite collection and inspection device. Using the measuring dispenser, the user need only dispense a required amount of sample (e.g., urine, suspected of containing a target antigen) in the vial of the lyophilized product containing the conjugates. The mixture may then be allowed to incubate for a prescribed period of time at room temperature. The resultant immunocomposite (if the test is positive) may then be collected using the collection device and the collected mass may be inspected for the color characteristic of the metal sol particle.

The simplicity of such diagnostic kit stems in part from the fact that all of the desirable or required additives for the reaction procedure have been incorporated into the lyophilized product mixture containing the conjugates. In this regard, it has been discovered that normally liquid organic components such as binding enhancing agents to enhance formation of immune complexes and detergents to suppress non-specific interactions may be distributed homogeneously throughout the lyophilized mixture and prevented from agglomeration at room temperature simply by incorporating a sugar comprising dextrin or trehalose into the solution prior to lyophilization. Upon lyophilization the sugar has the ability to prevent agglomeration of the organic component and thus to maintain the homogeneity of the mixture and thereby facilitate storage and shelf life of the mixture and the eventual dispersion of the mixture in an aqueous medium for conduct of the immunoassay procedure. As a result of the incorporation of the sugar in the lyophilized mixture, the immunoreactive components and the liquid organic components remain distributed homogeneously throughout the mixture during storage. As a result of the presence of the sugar and the resultant even distribution of the organic liquids in the lyophilized mixture, the reactivity, binding specificity and avidity of the immunoreactive component are retained and the lyophilized mixture retains its ability to be readily dispersed upon subsequent contact with an aqueous system.

A normally liquid organic component which desirably is included in the lyophilized mixture comprises a binding enhancing agent capable of accelerating formation of immune complexes from the conjugates. The binding enhancing agent may preferably be a non-ionic water soluble polymer such as polyethylene glycol, polyvinyl alcohol, polyvinyl pyrrolidone or dextran. Polyethylene glycol has found to be the optimum binding enhancing agent for utilization in the sol capture procedures of US 4 859 612 and AU-B-30268/89, which are discussed earlier in this specification and in enzyme assays.

As set forth above, the lyophilized mixture may preferably include all of the ingredients which desirably are present in the reaction system for the immunoassay. Thus, the lyophilized mixture may include buffer components selected to provide a pH of about 7.8 to 8.2 in the reaction milieu. Preferred buffer components, particularly for purposes of the sol capture procedures, include Tris base. Additionally, the system may advantageously include chelating agents such as EDTA disodium to tie up the divalent cations often present in urine. Other chelating agents such as ethylene diamine tetraacetic acid (EDTA), citric acid, tartaric acid, glucuronic acid, saccharic acid or suitable salts of these acids are enumerated in U.S. Patent 4,228,240 and could possibly be substituted for EDTA disodium. However, in the context of the present invention, EDTA disodium is preferred for use with urine test samples.

In accordance with the invention it has been found that oligosaccharides, preferably containing disaccharides (but not sucrose) and more preferably containing dextrin or trehalose sugars are important components in the lyophilization mixture for achieving the benefits of the present invention. Thus, when such sugars are present in the mixture in sufficient quantity, agglomeration of the binding enhancing agent and/or the surfactant is prevented and thus the homogeneity of the mixture is maintained. Accordingly, storage and shelf life of the mixture and the eventual dispersion of the mixture into an aqueous medium for conduct of an immunoassay procedure are facilitated.

The criticality associated with the selection of the specific class of sugars is surprising. It has been determined that the appropriate class of sugars includes members that exhibit a number of unique properties simultaneously. The appropriate sugars must be rapidly soluble in water, preferably dissolving in less than one minute. The sugars must also have the property that provides a visually homogeneous, stable solid mixture, i.e., a homogeneous stable matrix, of the lyophilized product containing the conjugates. Discovery of suitable sugars having the requisite combination of properties was difficult. Most sugars and also sucrose have been found to be unsatisfactory because they do not produce a homogeneous stable, solid mixture, i.e., a stable homogeneous matrix upon lyophilization, but instead produce lyophilized mixtures having concentration gradients for the individual components. The concentration gradients are believed to be the result of the agglomeration of the organic components at room temperature due to the fact that these components are normally liquid at room temperature. Such agglomeration results in the destruction of the homogeneity of the mixture and the resultant production of concentration gradients therein. The concentration gradients tend to have a deleterious effect on the conjugated antibodies caused by a more direct and concentrated exposure of the same to the agglomerated liquid globules in the lyophilized product. A suitable commercial product consisting of dextrins is available under the trade name MALTRIN™ from Grain Processing Corp.

The surface active agent (surfactant) for use in the mixture, and which may often be characterized as a detergent, may be selected from the class of water soluble non-ionic surface active agents. The surfactant may be selected from a wide variety of soluble non-ionic surface active agents. However, it has been determined that the most suitable surfactants are generally commercially available under the IGEPAL™ trade name from GAF Company. The IGEPAL™ liquid non-ionic surfactants are polyethylene glycol p-isooctylphenyl ether compounds and are available in various molecular weight designations, for example, IGEPAL™ CA720, IGEPAL™ CA630, and IGEPAL™ CA890. Other suitable non-ionic surfactants include those available under the trade name TETRONIC™ 909 from BASF Wyandotte Corporation. This material is a tetrafunctional block copolymer surfactant terminating in primary hydroxyl groups. Other suitable non-ionic surfactants are available under the VISTA ALPHONIC™ trade name from Vista Chemical Company and such materials are ethoxylates that are non-ionic biodegradables derived from linear primary alcohol blends of various molecular weights. Such non-ionic surfactants are most suitable for the purposes of the invention because they provide an appropriate amount of detergency for the assay without having a deleterious effect on the conjugate. In particular such surfactants are included in the reaction mixture for the purpose of suppressing non-specific interactions among the various ingredients of the immunoassay reaction system, and in this connection IGEPAL™ CA720 is a particularly preferred surfactant or detergent.

In view of the extreme sensitivity of the immunologic components to both environmental conditions and chemical environments, the difficulty of achieving a lyophilized product having the properties described herein should be clear. By the inclusion of the surfactant in the lyophilized product, the need for including a detergent as a separate kit component is eliminated.

The prior art teaches that surfactants such as detergents should normally be kept separated from immunoreactants. This is due to the fact that immunoreactants often are sensitive to detergents and surfactants and exposure to such materials often effects the binding properties of the immunoreactants. The same is true of binding enhancing agents such as polyethylene glycol, that is, exposure of immunoreactants to polyethylene glycol may effect the binding properties of the immunoreactant. However, in accordance with the present invention, it has been bound that the presence of a sugar comprising dextrin or trehalose in the lyophilized mixture eliminates or at least minimizes the effect of such ingredients on the binding properties of the immunoreagents. This may be the result of the property of the sugar which prevents agglomeration of the organic component and keeps it distributed homogeneously throughout the mixture in essentially a monodispersed form. Thus, the presence of the sugar may effectively retard contact between the liquid organic components and the immunoreactive components which are distributed homogeneously throughout the lyophilized mixture. In any event, it has been a surprising discovery that when a sugar comprising dextrin or trehalose is present in the lyophilized mixture in sufficient quantity, agglomeration of the organic component is prevented and the homogeneity of the mixture is maintained whereby storage and shelf life of the mixture and the eventual dispersion of the mixture in an aqueous medium for conduct of the immunoassay procedure are each facilitated. Additionally, the immunoreactive components distributed homogeneously throughout the lyophilized mixture do not lose their binding properties even though the mixture may be exposed to hot ambient conditions and stored for substantial periods of time.

The present invention provides a lyophilized mixture suitable for use with the sol capture assays described in US 4 859 612 and AU-B-30268/89, which are discussed earlier in this specification. Additionally, the lyophilized mixture of the present invention is useful in connection with ELISA type immunoassays. These assays are advantageously employed for the detection of particular antigens or antibodies which may be present in unknown concentrations in test samples. The immunoassay with which the lyophilized mixtures of the present invention may be employed are applicable to the measurement of a wide variety of specific antigens and antibodies and they have been found to have particular utility in home diagnostic kits for detection of antigens such as human chorionic gonadotropin hormone (hCG) which is present in the urine of pregnant women. The lyophilized mixture of the present invention may also be utilized in connection with assays for the detection of Neisseria gonorrhea, the bacteria causing gonorrhea, also called gonococcus (GC) and human luteinizing hormone (hLH), a hormone that is present in female urine at the time of ovulation.

As set forth above, the present invention provides a lyophilized mixture which has great utility in connection with the sol capture procedures described in US 4 859 612 and AU-B-30268/89, which are discussed earlier in this specification. Thus, the lyophilized mixture includes two dispersible immunoreactive components, one of which is an antibody-gold sol particle conjugate and the other of which is an antibody-solid carrier particle conjugate. The preparation of such conjugates is fully described in US 4 859 612, which is discussed earlier in this specification. In this preferred embodiment of the invention, the lyophilization mixture also should contain both a polyethylene glycol binding enhancing agent and a polyethylene glycol p-isooctylphenyl ether surface active agent to suppress non-specific interactions between the various components of the reaction mixture. Preferably the preferred lyophilized mixture may also contain a Tris base buffering agent to control pH, and an EDTA disodium salt as a chelating agent to tie up divalent cations from human urine. Thimerosal may be included as an anti-bacterial agent to improve shelf life prior to freeze drying and in this sense it should be recognized that the thimerosal is present in the freeze dried product not so much because of its effect on the lyophilized mixture but more so because of its effect on the material prior to freeze drying.

In accordance with the invention, to prepare an appropriate lyophilized mixture for use in a gold sol capture procedure, the solution to be subjected to lyophilization may contain materials suitable for detecting the presence of hCG using assay procedures which are generally the same as those described in Examples IV(f) and (g) of US 4 859 612, which is discussed earlier in this specification. The antibody coated gold sol particles may be prepared essentially as set forth in the prior application and preferably the gold sol particles should have a diameter of approximately 30nm. (See, G. Frens, Nature 241, 20-22, (1973)). The preferred antibody for coating the gold sol particles is the 2G9 antibody described in US 4 859 612, which is discussed earlier in this specification. The antibody coated gold sol particles may be processed as set forth in US 4 859 612, which is discussed earlier in this specification, to produce a final product which comprises a suspension of the gold labelled probe particles and which may then be used as the labelled component in the preferred immunoassay of the invention. Thus, the labelled component comprises the 2G9 antibody conjugated to an appropriate amount of 30nm gold sol particles.

In the preferred form of the invention, the assay procedure further involves the use of a solid phase component which comprises the coupling product of a second antibody and a solid phase particle. The second antibody is also an antibody to hCG and the same is referred to as 2B2 antibody in US 4 859 612, which is discussed earlier in this specification. In accordance with this preferred aspect of the invention the 2B2 antibody and the 2G9 antibody are specifically immunoreactive with respect to different sites or epitopes on the hCG molecule. That is to say, 2B2 antibody reacts with the hCG molecule at one specific epitopic site, while the 2G9 antibody reacts with the hCG molecule at a different, spatially removed specific epitopic site. Thus, when the test is positive, a composite made up of the gold sol particle, the 2G9 antibody, the hCG molecule, the 2B2 antibody and the solid phase particle is formed. Such composite is sufficiently large to be captured on a filter element where the inherent and distinctive coloration of the bound gold sol particles may be visually observed.

The 2B2 antibody-solid carrier particle conjugate for use in connection with the preferred form of the invention may be prepared using any of the various methods and materials disclosed and described in US 4 859 612, which is discussed earlier in this specification. Thus, 2B2 antibody-latex probe particles are prepared by conjugating the 2B2 antibodies to 0.99µ carboxylated modified latex particles.

In accordance with the present invention, an admixture of freeze dried antibody-latex particle conjugates and freeze dried antibody-gold sol particle conjugates may be provided in a single test container (or vial) containing an amount of each conjugate needed for conducting a single test. The admixture of freeze dried antibody-particle conjugates may be prepared by forming a single dispersion containing both species of conjugates and freeze drying the conjugates together. This provides a simplified kit for commercial purposes. Additionally, the other ingredients to be included in accordance with the immunoassay may be incorporated in the lyophilized mixture in the test container at this time.

Each individual test should preferably contain gold probe particles consisting of about 2.75µg of 2G9 antibody conjugated to about 0.27 OD₅₃₃ₙₘ units of 30nm gold sol particles, latex probe particles consisting of about 6.0µg of 2B2 antibody conjugated to about 1.0 OD₅₀₀ₙₘ units of 0.99µ carboxylated modified latex particles. To prepare a lyophilized mixture from such immunoreactive components, a single aqueous system is provided and which includes the various components necessary or desirable in the immunoassay procedures. Thus, 500ml of an aqueous system to be subjected to freeze drying is prepared. The system preferably contains 2.75mg of the 2G9 antibody conjugated to gold sol particles; 6.0mg of the 2B2 antibody conjugated to carboxylated modified latex particles; 10.0mg of polyethylene glycol (20M) as a binding enhancing agent; 48mg of thimerosal; 5.8gms of Tris base buffer; 48gms of Maltrin; 1.44gms of EDTA disodium salt and 0.144ml of IGEPAL™ CA720. The liquid system comprises a solution of soluble materials and a dispersed suspension of non soluble materials such as the antibody-particle conjugates. The resultant dispersion, which contains sufficient ingredients for the conduct of approximately 1000 test procedures, is distributed equally among 1000 glass lyophilization vials and subjected to conventional freeze drying procedures.

The lyophilization cycle which has been found to be preferable requires the initial freezing of the aqueous lyophilization dispersion containing the conjugate. The dispersion is frozen in a conventional lyophilization chamber maintained at a temperature range between about -40°C and -35°C for at least 2 hours at atmospheric conditions. The frozen lyophilization dispersion containing the antibody-particle conjugates is subjected to a vacuum of about 10 to 50 milli torr over a 24 hour period. During this 24 hour cycle the temperature in the lyophilization chamber is adjusted to incremental levels while the high vacuum conditions are maintained at about 20 to 50 milli torr. Typical temperature levels employed during the lyophilization cycle are -10°C for 18 hours; and +25°C for about 7 to 10 hours until the product reaches about 25°C. The lyophilization chamber is then partially filled with nitrogen and the vials containing the lyophilized product are stoppered. During the lyophilization cycle, the liquid initially contained in the lyophilization dispersion evaporates. The resulting lyophilized product containing antibody-particle conjugates comprises a lyophilized mixture and the various ingredients thereof are distributed homogeneously throughout the mixture.

As desired, the dispersion to be subjected to freeze drying may contain other ingredients which are desirably present during the conduct of the immunoassay. Thus, a green dye as described in AU-B-30268/89, which is discussed earlier in this specification, may be included in the dispersion to be subjected to freeze drying whereby the green dye will be present in the lyophilized mixture.

The lyophilization vials subjected to lyophilization, as set forth above, each contain a lyophilized mixture for use in an immunoprocedure. The mixture comprises two dispersible immunoreactive components distributed homogeneously throughout the mixture. One of the dispersible immunoreactive components is the 2B2 antibody conjugated to 0.99µ carboxylated modified latex particles, and the other of the immunoreactive components comprises the 2G9 antibody conjugated to 30nm gold sol particles. The lyophilized mixture in the lyophilization vial also includes two normally liquid organic components, the first of which comprises the IGEPAL™ CA720 detergent and the other of which comprises polyethylene glycol (20M). As set forth above, the IGEPAL™ component is included to inhibit non-specific interactions between the components of the system. And the polyethylene glycol is present to enhance the binding activity of the immunoreactants. The amounts of these materials is not a critical feature of the present invention; however, the same need to be present in amounts which are deemed either necessary or desirable to achieve the sought after results, and suffice it to say, the present invention provides a mechanism for diminishing and/or eliminating the undesirable results which otherwise might be experienced when the liquid organic components are used.

The lyophilized mixture in the lyophilization vial also contains the sugar. Each vial contains approximately 48µg of Maltrin, an amount determined empirically to prevent agglomeration of the 0.144µl of IGEPAL™ CA720 and the 10.0µg of PEG present in each lyophilization vial. However, it should be recognized that the amount of the sugar is not critical other than that the same must be present in an amount which is sufficient to prevent agglomeration of the normally liquid components whereby the homogeneity of the mixture is maintained and the storage and shelf life and eventual dispersion of the mixture in an aqueous medium are both facilitated. As will be appreciated by those of ordinary skill in the art to which the present invention pertains, the optimum amount of the sugar to be included in any given mixture of ingredients will depend on a number of unrelated phenomena and must generally be determined empirically. In this regard, the agglomeration of the liquid organic components will simply cause the lyophilized mixture to have paste like rather than particulate characteristics and the determination of a correct amount of sugar to achieve the desired particulate characteristics will not be difficult for the ordinary experimenter in the relevant art.

The lyophilized mixture of the present invention has been utilized successfully in the performance of the immunoassays described in US 4 859 612 and AU-B-30268/89, which are discussed earlier in this specification. To conduct such immunoassays, the lyophilized mixture is simply contacted with 0.4ml of urine and the lyophilized mixture quickly disperses in the urine in a matter of a few seconds.

## Claims

1. A lyophilized mixture for use in an immunoassay procedure comprising:
at least one dispersible immunoreactive component distributed homogeneously throughout said mixture;
at least one organic component distributed homogeneously throughout said mixture, said organic component normally being a liquid at the conditions under which the mixture is stored or retained prior to use and having a property which enhances the performance of the immunoassay by its presence; and
a sugar comprising dextrin or trehalose, said dextrin or trehalose being present in said mixture in sufficient quantity to prevent agglomeration of the organic component and thus maintain the homogeneity of the mixture to thereby facilitate storage and shelf life of the mixture and the eventual dispersion of the mixture in an aqueous medium for conduct of the immunoassay procedure.

2. A lyophilized mixture as set forth in claim 1, wherein said immunoreactive component comprises an antibody conjugate, optionally an antibody-enzyme conjugate.

3. A lyophilised mixture as set forth in claim 1 or claim 2, wherein said organic component comprises a binding enhancing agent, optionally a non-ionic, water soluble polymer, and/or wherein said organic component comprises a surfactant.

4. A lyophilized mixture as set forth in claim 3, wherein said polymer comprises polyethylene glycol, polyvinyl alcohol, polyvinyl pyrrolidone, or dextran.

5. A lyophilized mixture as set forth in claim 3 or claim 4, wherein said surfactant comprises a water soluble non-ionic surface active agent, optionally a polyethylene glycol p-isooctylphenyl ether compound.

6. A lyophilized mixture as set forth in claim 2, wherein either:
(a) said antibody conjugate comprises an antibody-gold sol particle conjugate or wherein said antibody conjugate comprises and antibody-solid carrier particle conjugate, optionally said solid carrier particle comprising a latex particle; or
(b) wherein said mixture includes at least two immunoreactive components and wherein one of the immunoreactive components comprises an antibody-gold sol conjugate and the other immunoreactive component comprises an antibody-solid carrier particle, optionally said solid carrier particle comprising a latex particle.

7. A lyophilized mixture as set forth in claim 6, wherein said mixture includes at least two said liquid organic components and wherein one of the organic components comprises a binding enhancing agent and the other organic component comprises a surfactant, optionally said binding enhancing agent comprising a polyethylene glycol and said surfactant comprising a polyethylene glycol p-isooctylphenyl ether compound.

8. A mixture as set forth in claim 6 or claim 7 and which further comprises the specific feature(s) of one or more of claims 3, 4 or 5.

9. A method of lyophilizing an immunoreactive component for use in an immunoassay, which method comprises forming a mixture of ingredients as defined in any one of claims 1 to 5 or 6 to 8 including said component and lyophilizing the resulting mixture.

10. A method of forming a storage stable lyophilized composition readily reconstitutable for use in an immunoassay and comprising at least one organic component normally being a liquid at the conditions under which the mixture is stored or retained prior to use and having a property which enhances the performance of the immunoassay by its presence, which method comprises incorporating a sugar comprising dextrin or trehalose in a mixture including said organic component and at least one dispersible immunoreactive component distributed homogenously throughout said mixture, and lyophilizing said mixture.

11. The use of a lyophilized mixture as defined in any one of claims 1 to 5 or 6 to 8 in an immunoassay procedure.

## Patentansprüche

1. Ein lyophilisiertes Gemisch zur Verwendung in einem Immunnachweisverfahren, umfassend:
mindestens eine dispergierbare immunreaktive Komponente homogen verteilt innerhalb des Gemisches,
mindestens eine organische Komponente homogen verteilt innerhalb des Gemisches, wobei die organische Komponente unter den Bedingungen, bei denen das Gemisch vor Gebrauch gelagert oder aufbewahrt wird, normalerweise eine Flüssigkeit ist und eine Eigenschaft besitzt, die die Leistung des Immunnachweises durch ihre Anwesenheit steigert, und
einen Zucker, umfassend Dextrin oder Trehalose, wobei Dextrin oder Trehalose in dem Gemisch in ausreichender Menge vorhanden ist, um eine Agglomeration der organischen Komponente zu verhindern und so die Homogenität des Gemisches aufrechtzuerhalten, um damit die Lagerung und die Lagerbeständigkeit des Gemisches und die schließliche Dispersion des Gemisches in einem wäßrigen Medium zur Durchführung des Immunnachweisverfahrens zu erleichtern.

2. Ein lyophilisiertes Gemisch nach Anspruch 1, in dem die immunreaktive Komponente ein Antikörper-Konjugat, gegebenenfalls ein Antikörper-Enzym-Konjugat, umfaßt.

3. Ein lyophilisiertes Gemisch nach Anspruch 1 oder 2, in dem die organische Komponente ein bindungssteigerndes Mittel, gegebenenfalls ein nichtionisches, wasserlösliches Polymer umfaßt, und/oder in dem die organische Komponente ein Tensid umfaßt.

4. Ein lyophilisiertes Gemisch nach Anspruch 3, in dem das Polymer Polyethylenglykol, Polyvinylalkohol, Polyvinylpyrrolidon oder Dextran umfaßt.

5. Ein lyophilisiertes Gemisch nach Anspruch 3 oder 4, in dem das Tensid ein wasserlösliches, nichtionisches grenzflächenaktives Mittel ist, gegebenenfalls eine Polyethylenglykol-p-isooctylphenylether-Verbindung.

6. Ein lyophilisiertes Gemisch nach Anspruch 2, in dem entweder:
(a) das Antikörper-Konjugat ein Antikörper-Gold-Solpartikel-Konjugat ist, oder in dem das Antikörper-Konjugat ein Antikörper-Feststoffträgerpartikel-Konjugat umfaßt, wobei gegebenenfalls das Feststoffträgerpartikel ein Latexpartikel umfaßt, oder
(b) das Gemisch mindestens zwei immunreaktive Komponenten beinhaltet und wobei eine der immunreaktiven Komponenten ein Antikörper-Gold-Sol-Konjugat und die andere immunreaktive Komponente ein Antikörper-Feststoffträgerpartikel umfaßt, wobei gegebenenfalls das Feststoffträgerpartikel ein Latexpartikel umfaßt.

7. Ein lyophilisiertes Gemisch nach Anspruch 6, in dem das Gemisch mindestens zwei der flüssigen organischen Komponenten enthält und wobei eine der organischen Komponenten ein bindungssteigerndes Mittel und die andere organische Komponente ein Tensid umfaßt, wobei gegebenenfalls das bindungssteigernde Mittel ein Polyethylenglykol und das Tensid eine Polyethylenglykol-p-isooctylphenylether-Verbindung umfaßt.

8. Ein Gemisch nach Anspruch 6 oder 7 und weiter umfassend das (die) spezifische(n) Merkmal(e) von einem oder mehreren der Ansprüche 3, 4 oder 5.

9. Ein Verfahren zur Lyophilisierung einer immunreaktiven Komponente zur Verwendung in einem Immunnachweis, wobei das Verfahren das Bilden eines Gemisches aus Bestandteilen, gemäß einem der Ansprüche 1 bis 5 oder 6 bis 8, beinhaltend die Komponente und das Lyophilisieren des erhaltenen Gemisches, umfaßt.

10. Ein Verfahren der Bildung einer lagerstabilen, leicht rekonstituierbaren lyophilisierten Zusammensetzung zur Verwendung in einem Immunnachweis und umfassend mindestens eine organische Komponente, die unter den Bedingungen, bei denen das Gemisch vor Gebrauch gelagert oder aufbewahrt wird, normalerweise eine Flüssigkeit ist und eine Eigenschaft besitzt, die die Leistung des Immunnachweises durch ihre Anwesenheit steigert, wobei das Verfahren das Einverleiben eines Zuckers umfassend Dextrin oder Trehalose in ein Gemisch der organischen Komponente und mindestens einer dispergierbaren immunreaktiven Komponente homogen verteilt innerhalb des Gemisches und das Lyophilisieren des Gemisches umfaßt.

11. Die Verwendung eines lyophilisierten Gemisches gemäß einem der Ansprüche 1 bis 5 oder 6 bis 8 in einem Immunnachweisverfahren.

## Revendications

1. Mélange lyophilisé destiné à être utilisé dans un test immunologique comprenant:
au moins un composant immunoréactif dispersible distribué de manière homogène dans ledit mélange;
au moins un composant organique distribué de manière homogène dans ledit mélange, ledit composant organique étant normalement un liquide dans les conditions dans lesquelles le mélange est stocké ou conservé avant emploi et possèdant une propriété telle que la performance du test immunologique est accrue par sa présence; et
un sucre comprenant la dextrine ou le tréhalose, ladite dextrine ou ledit tréhalose étant présent dans ledit mélange en quantité suffisante pour empêcher l'agglomération du composant organique et conserver ainsi l'homogénéité du mélange afin de faciliter le stockage et la conservation du mélange et enfin sa dispersion dans un milieu aqueux afin de réaliser le test immunologique.

2. Mélange lyophilisé selon la revendication 1, où ledit composant immunoréactif comprend un anticorps conjugué, éventuellement un anticorps conjugué à une enzyme.

3. Mélange lyophilisé selon la revendication 1 ou la revendication 2, où ledit composant organique comprend un agent facilitant la liaison, éventuellement un polymère hydrosoluble non-ionique, et/ou où ledit composant organique comprend un agent tensioactif.

4. Mélange lyophilisé selon la revendication 3, où ledit polymère comprend le polyéthylène glycol, l'alcool polyvinylique, le polyvinyle pyrrolidone ou le dextrane.

5. Mélange lyophilisé selon la revendication 3 ou 4, où ledit agent tensioactif comprend un agent tensioactif non-ionique hydrosoluble, éventuellement un composé d'éther de polyéthylène glycol p-isooctylphénylique.

6. Mélange lyophilisé selon la revendication 2, dans lequel:
(a) soit ledit anticorps conjugué comprend un anticorps conjugué à une particule d'or colloïdal ou dans lequel ledit anticorps conjugué comprend un anticorps conjugué à une particule solide servant de véhicule, ladite une particule solide servant de véhicule comprenant éventuellement une particule de latex;
(b) soit ledit mélange comprend au moins deux composants immunoréactifs et dans lequel l'un des composants immunoréactifs comprend un anticorps conjugué à une particule d'or colloïdal et l'autre composant immunoréactif comprend un anticorps conjugué à une particule solide servant de véhicule, ladite une particule solide servant de véhicule comprenant éventuellement une particule de latex.

7. Mélange lyophilisé selon la revendication 6, dans lequel ledit mélange inclut au moins deux desdits composants organiques liquides et dans lequel l'un des composants organiques comprend un agent favorisant la liaison et l'autre composant organique comprend un agent tensioactif, ledit agent favorisant la liaison comprenant éventuellement un polyéthylène glycol et ledit agent tensioactif comprenant un composé d'éther de polyéthylène glycol p-isooctylphénylique.

8. Mélange selon la revendication 6 ou la revendication 7, qui comprend en outre les caractéristiques spécifiques d'une ou de plusieurs des revendications 3, 4 ou 5.

9. Procédé de lyophilisation d'un composant immunoréactif destiné à être utilisé dans un test immunologique, qui comprend la préparation d'un mélange d'ingrédients selon l'une des revendications 1 à 5 ou 6 à 8 comprenant ledit composant et la lyophilisation du mélange résultant.

10. Procédé de préparation d'une composition stable lyophilisée facile à reconstituer destinée à être utilisée dans un test immunologique et comprenant au moins un composant organique qui est normalement un liquide dans les conditions dans lesquelles le mélange est stocké ou conservé avant emploi et possèdant une propriété telle que la performance du test immunologique est accrue par sa présence, lequel procédé comprend l'incorporation d'un sucre comprenant la dextrine ou le tréhalose dans un mélange incluant ledit composant organique et au moins un composant immunoréactif dispersible distribué de manière homogène dans ledit mélange et la lyophilisation dudit mélange.

11. Utilisation d'un mélange lyophilisé comme défini selon l'une des revendications 1 à 5 ou 6 à 8 dans la procédure d'un test immunologique.
